(19) Europäisches Patentamt
European Patent Office
Office européen des brevets

(11) Publication number: **0 102 721**
**B1**

(12) **EUROPEAN PATENT SPECIFICATION**

(45) Date of publication of patent specification: **21.11.85**

(51) Int. Cl.⁴: **C 07 H 17/08**, C 07 D493/22, A 61 K 31/70, A 61 K 31/355, A 01 N 43/24 // (C07D493/22, 313:00, 311:00, 311:00, 307:00)

(21) Application number: **83304145.2**

(22) Date of filing: **18.07.83**

(54) **Milbemycin D derivatives, their preparation and compositions containing them.**

(30) Priority: **23.07.82 JP 128472/82**

(43) Date of publication of application: **14.03.84 Bulletin 84/11**

(45) Publication of the grant of the patent: **21.11.85 Bulletin 85/47**

(84) Designated contracting states: **BE CH DE FR GB IT LI NL**

(56) References cited:
**EP-A- 0 074 758**
**GB-A- 2 056 986**

(73) Proprietor: **SANKYO COMPANY LIMITED**
**No. 1-6, 3-chome Nihonbashi Honcho Chuo-ku**
**Tokyo (JP)**

(72) Inventor: **Ide, Junya c/o Chemical Research Lab.**
**Sankyo Co., Ltd. 2-58, 1-chome, Hiromachi**
**Shinagawa-ku Tokyo (JP)**
Inventor: **Muramatsu, Shigeki Chemical Research Laboratories**
**Sankyo Co., Ltd. 2-58, 1-chome, Hiromachi**
**Shinagawa-ku Tokyo (JP)**
Inventor: **Nakada, Yasuo c/o Patent Department**
**Sankyo Co., Ltd. 2-58, 1-chome, Hiromachi**
**Shinagawa-ku Tokyo (JP)**
Inventor: **Kitano, Noritoshi Biological Research Laboratories**
**Sankyo Co., Ltd. 2-58, 1-chome, Hiromachi**
**Shinagawa-ku Tokyo (JP)**
Inventor: **Yajima, Masaru Product Development Research Lab.**
**Sankyo Co., Ltd. 2-58, 1-chome, Hiromachi**
**Shinagawa-ku Tokyo (JP)**
Inventor: **Ohkura, Yoshinori Product Development Res. Lab.**
**Sankyo Co., Ltd. 2-58, 1-chome, Hiromachi**
**Shinagawa-ku Tokyo (JP)**

(74) Representative: **Gibson, Christian John Robert et al**
**Marks & Clerk 57/60 Lincoln's Inn Fields**
**London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 102 721 B1

Jouve, 18, rue St-Denis, 75001 Paris, France

**Description**

The present invention relates to a series of new derivatives of the compound known as milbemycin D, to a process for their preparation and to pharmaceutical, veterinary, agricultural and horticultural compositions containing them.

Milbemycin D was disclosed in U.S. Patent No. 4.346.171, where it was referred to as «compound B-41D», and may be represented by the formula (I) :

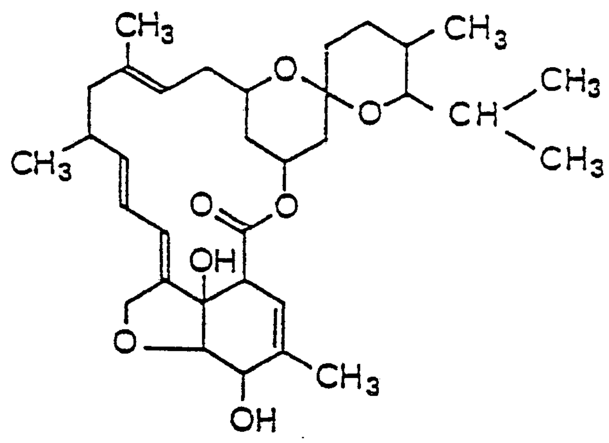

(I)

Milbemycin D was found to have valuable anthelmintic and acaricidal activities and was prepared by cultivation of the Streptomyces strain B-41-146, which had been deposited at the Fermentation Research Institute. Agency of Industrial Science and Technology, Ministry of International Trade and Industry, Japan, whence it is available under the accession number FERM-1438.

We have now discovered a series of derivatives of milbemycin D having good anthelmintic, acaricidal and insecticidal activities, which are, in certain test systems, significantly better than the corresponding activities of milbemycin D itself.

The compounds of the present invention are the 5-milbemycin D half-esters of dibasic acids and salts thereof. These half-esters may be represented by the formula (II) :

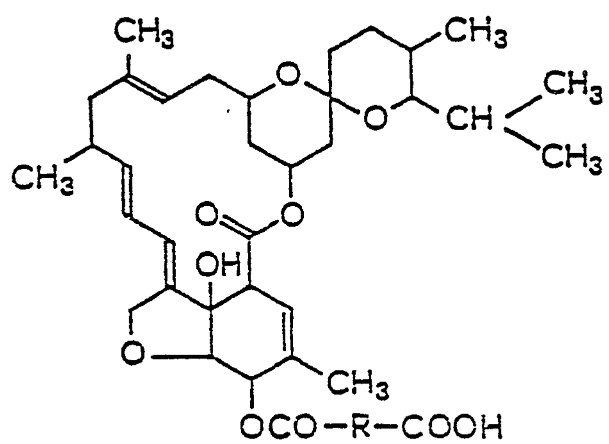

(II)

in which R represents a straight chain alkylene group having from 1 to 4 carbon atoms, a vinylene group or a group of formula

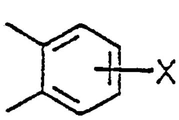

in which X represents a hydrogen atom or an alkyl group having from 1 to 6 carbon atoms.

The invention further provides a process for producing the compounds of the invention by reacting milbemycin D with a dibasic acid of formula HOOC—R—COOH (in which R is a defined above) or with a functional equivalent thereof, one carboxy group in said acid or equivalent being optionally protected, if necessary removing the protecting group and, if necessary, salifying the product.

The invention also provides an acaricidal, anthelmintic and insecticidal composition for pharmaceutical, veterinary, agricultural or horticultural use and comprising, as active ingredient, at least one of the compounds of the invention in admixture with a carrier or diluent.

The invention also provides a method of protecting plants or non-human animals from damage by acarids, helminths or insects, by applying to said animals, seeds, said plants or a locus including the same at least one compound of the present invention.

In the compounds of formula (II), where R represents an alkylene group, it may be a methylene, ethylene, trimethylene or tetramethylene group, more preferably an ethylene or trimethylene group.

Where R represents a substituted or unsubstituted phenylene group of formula :

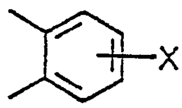

and X represents an alkyl group, this may be a straight or branched chain group having from 1 to 6, more preferably from 1 to 4, carbon atoms, such as a methyl, ethyl, propyl, butyl or isobutyl group, more preferably a methyl group. However, X most preferably represents a hydrogen atom.

Compounds of formula (II) are able to form salts, for example : alkali metal salts, such as lithium, sodium or potassium salts ; alkaline earth metal salts, such as calcium or barium salts ; other metal salts, such as magnesium salts ; salts with basic amino acids, such as lysine or arginine ; ammonium salts ; and salts with organic amines, such as cyclohexylamine, diisopropylamine, triethylamine, aniline, N,N-dimethyl-aniline or pyridine. Of these, the sodium and potassium salts are particularly preferred since water-soluble salts, such as the sodium salt, are well absorbed into the animal body.

The most preferred compounds of the invention are compounds of formula (II) in which R represents an ethylene, trimethylene, vinylene or o-phenylene group and their sodium and potassium salts.

Compounds of formula (II) may be prepared by reaction of milbemycin D, having the aforementioned formula (I), with a dibasic acid of formula HOOC—R—COOH (in which R is as defined above) or with a functional equivalent of said acid.

Suitable functional equivalents include any of those commonly used for conventional esterification reactions, such as the acid halides (preferably the mono acid halide), mixed anhydrides with monocarboxylic acids and internal anhydrides. Preferred acids and functional equivalents are compounds of formula (III) :

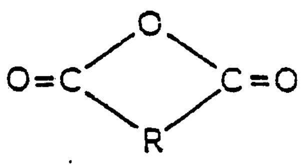

(III)

i. e. the internal anhydrides, and compounds of formula (IV) :

Y—CO—R—COOH (IV)

(in which : R is as defined above ; Y represents a hydroxy group, a halogen atom or a $C_2$-$C_5$ alkylcarboxy group ; and the group —COOH is optionally protected).

Where the functional equivalent is a mono acid halide, the remaining carboxyl group of the dicarboxylic acid is preferably protected with a conventional protecting group, such as an alkyl (e. g. t-butyl), aralkyl (e. g. benzhydryl) or haloalkyl (e. g. 2,2,2-trichloroethyl) group. Of the dicarboxylic acids and functional equivalents thereof, we prefer to use either the mono acid halide in which the remaining carboxyl group has been protected or the internal anhydride, most preferably the internal anhydride, which may be represented by the formula (III).

The reaction is preferably effected in the presence of a base and of an inert solvent. Examples of suitable bases include organic amines, such as triethylamine, pyridine, 4-dimethylaminopyridine, N,N-dimethylaniline, 1,5-diazabicyclo [4,3,0] nonene-5 or 1,8-diazabicyclo-[5,4,0] undecene-7. There is no particular limitation on the nature of the solvent employed in this reaction, provided that it has no adverse effect on the reaction. Suitable solvents include : hydrocarbons, such as hexane, benzene, toluene or xylene ; ethers, such as diethyl ether or tetrahydrofuran ; and halogenated hydrocarbons, such as methylene chloride or chloroform.

In general, the reaction temperature may vary over a wide range, although a relatively low temperature is normally preferred, in order to minimise side reactions. Accordingly, a suitable temperature range is from − 10 to + 50 °C, more preferably about room temperature. At a reaction temperature within this range, the reaction will normally require a period of from 1 to 50 hours.

Where a mono acid halide whose carboxyl group is protected is employed as the functional

equivalent, the protecting group may be removed by conventional means, the precise reaction depending upon the nature of the group. For example, where the group is an alkyl or aralkyl group, this may be removed by treating the product with an acid or, where it is a haloalkyl group, it may be removed by reaction with zinc/acetic acid.

After completion of the reaction, the desired product may be recovered from the reaction mixture by conventional means, for example by pouring the reaction mixture into ice-water and then extracting the product with a water-immiscible organic solvent. After drying the organic extract and distilling off the solvent, the resulting residue can, if necessary, be further purified by such conventional techniques as recrystallisation and/or column chromatography.

Salts of the compounds of formula (II) can readily be prepared by reacting the compound of formula (II) with an appropriate alkali or base. For example, in the case of the preferred sodium and potassium salts, these may be prepared by reacting the compound with a hydroxide, carbonate or bicarbonate of sodium or potassium, preferably sodium or potassium bicarbonate.

The compounds of the invention have a strong acaricidal activity against, for example, adults, imagos and eggs of Tetranychus, Panonychus and rust mites, which are parasitic to fruit trees, vegetables and flowers. They are also active against Ixodidac, Dermanysside and Sarcoptidae, which are parasitic to animals. Further, they are active against exoparasites, such as Oestrus, Lucilia, Hypoderma, Gautrophilus, lice and fleas, which are parasitic to animals and birds ; domestic insects, such as cockroaches and houseflies ; and various harmful insects in agricultural and horticultural areas, such as aphids and larval Lepidoptera. They are also effective against Meloidogyne in the soil and Phizoglyphus. They are also effective against insects of the orders Coleoptera, Homoptera, Heteroptera, Diptera, Thysanoptera, Orthoptera, Anoplura, Siphonaptera, Mallophage, Thysanura, Isoptera, Psocoptera, and Hymenoptera.

The compounds of the invention equally can be used to control other plant-damaging insects, particularly insects that damage plants by eating them. The compounds can be used to protect both ornamental plants and productive plants, particularly cotton (e. g. against Spodoptera littoralis and Heliothis virescens), as well as vegetable crops (e. g. against Leptinotarsa decemlineata and Myzus persicae) and rice crops (e. g. against Chilo suppressalis and Laodelphax).

The activity of the compounds of the invention is pronounced, both systemically and by contact. Accordingly, the compounds are very effective against sucking insects, especially sucking insects of the order Homoptera and most particularly the family Aphididae (such as Aphis fabae, Aphis craccivora and Myzus persicae), which are difficult to control with known compositions.

Accordingly, the compounds of the invention can be used to treat all manner of plants (as well as the seeds from which such plants are grown and the environment containing such plants) to protect them from insects such as those exemplified above. Such plants include cereals (e. g. maize or rice), vegetables (e. g. potatoes or soybeans), fruits and other plants (e. g. cotton).

The compounds of the invention can similarly be used to protect animals from a variety of ectoparasites, by applying the compounds to the animals or to the animals' environment, e. g. livestock housing, animal boxes, abattoirs, pasture land and other grasslands, as well as to any other places liable to be infested. The compounds may also be applied to external parts of the animals, preferably before they are infested.

Moreover, the compounds of the invention are effective against various parasitical helminths. These parasites can attack livestock, poultry and pet animals (such as pigs, sheep, goats, cows, horses, dogs, cats and fowl) and can cause grave economic damage. Among the helminths, the nematodes in particular often cause serious infection. Typical genera of nematodes which are parasitic on these animals and against which the compounds of the invention are effective include :

Haemonchus,
Trichostrongylus,
Ostertagia,
Nematodirus,
Cooperia,
Ascaris,
Bunostomum,
Oesophagostomum,
Chabertia,
Trichuris,
Strongylus,
Trichonema,
Dictyocaulus,
Capillaria,
Heterakis,
Toxocara,
Ascaridia,
Oxyuris,
Ancylostoma,

Uncinaria,
Toxascaris and
Parascaris.

Certain species of the genera Nematodirus, Cooperia and Oesophagostomum attack the intestines, while certain species of the genera Haemonchus and Ostertagia parasitize the stomach, and parasites belonging to the genus Dictyocaulus are found in the lungs. Parasites belonging to the families Filariidae and Setariidae are found in internal tissues and organs, for example, the heart, the blood vessels, the subcutaneous tissues and the lymphatic vessels. The compounds of the invention are active against all these parasites.

The compounds of the invention are also effective against parasites which infect humans. Typical of the parasites which may most commonly be found in the digestive tracts of human beings are parasites of the genera Ancylostoma, Necator, Ascaris, Strongyloides, Trichinella, Capillaria, Trichuris and Enterobius. The compounds are also active against parasites of the genera Wuchereria, Brugia, Onchocerca and Loa of the family Filariidae (which are found in blood, tissues and organs other than the digestive tract and are medically important), parasites of the genus Dracunculus and parasites of the genera Strongyloides and Trichinella, which especially infect the exointestinal canal.

The form of the compositions of the invention and the nature of the carriers or diluents employed in them will vary depending upon the intended use of the composition. For example, where the compounds of the invention are to be employed as anthelmintics, they are preferably administered orally, parenterally or topically and the form of compositions chosen will be appropriate to the intended route of administration.

For oral administration, the composition of the invention is preferably in the form of a liquid drink comprising a non-toxic solution or suspension, preferably aqueous, of the active compound in admixture with a suspending agent (such as bentonite), a wetting agent or other diluents. The drink, in general, also contains an anti-foaming agent. The active compound would normally be present in the drink in an amount of from 0.01 to 0.5% by weight, more preferably from 0.01 to 0.1% by weight.

Compositions for oral administration may also be in the form of dry solids, preferably in unit dosage form, such as capsules, pills or tablets containing the desired amount of the active compound. These compositions may be prepared by mixing the active compound uniformly with suitable diluents, fillers, disintegrators and/or binding agents, for example starch, lactose, talc, magnesium stearate and vegetable gum. The weight and contents of the preparation will vary widely, depending upon the nature of the animal to be treated, the degree of infection, the nature of the parasite and the body weight of the animal to be treated.

The compounds may also be administered as an additive to animal feedstuffs, in which case they may be dispersed uniformly in the feedstuffs, used as a top dressing or used in the form of pellets. The content of active compound in the feedstuff is preferably from 0.000 1 to 0.02 %, in order to achieve the desired anthelmintic activity.

For parenteral administration, the compound of the invention is preferably dissolved or suspended in a liquid vehicle, preferably a vegetable oil, such as peanut oil or cottonseed oil. Where the compound is a salt of a compound of formula (II), the liquid vehicle may be water or another aqueous medium. Depending upon the animal to be treated, the injection may be subcutaneous or into the proventriculus, a muscle or the trachea. Such preparations would normally contain the active compound at a concentration of from 0.05 to 50 % by weight.

The compounds of the invention may also be administered tropically in admixture with a suitable carrier, such as a hydrocarbon solvent. Such preparations would be applied directly to the outside of the animal by spraying (e. g. by a hand spray or in spray races), by dipping (e. g. in a plunge dip), by a pour-on solution or by manual methods (e. g. hand-dressing).

The dose of active compound may be varied, depending upon the nature of the animal to be treated, and the nature and degree of parasitic infection. However, best results for oral administration are achieved when the dose is from 0.01 to 100 mg, more preferably from 0.5 to 50 mg, per 1 kg body weight. The compound may be administered in a single dose or in divided doses for a relatively short period, such as from 1 to 5 days.

Where the composition of the invention is intended for agricultural or horticultural use, a variety of forms and formulations are possible. For example, it may be formulated as dusts, coarse dusts, soluble powders, microgranules, fine microgranules, wettable powders, dilute emulsions, emulsifiable concentrates, aqueous or oily suspensions or solutions (which may be directly sprayable or for dilution), aerosols or capsules in, for example, polymeric substances. The carrier employed may be natural or synthetic and organic or inorganic ; it is generally employed to assist the active compound to reach the substrate to be treated, and to make it easier to store, transport or handle the active compound. Solid, liquid and gaseous carriers may be employed, chosen from carriers well known in the art for use with compositions of this type.

Such formulations may be prepared by conventional means, e. g. by intimate mixing and/or grinding of the active ingredient(s) with the carrier or diluent, e. g. solvent, solid carrier or, optionally, surface-active agent.

Suitable solvents include : aromatic hydrocarbons, preferably the $C_8$ to $C_{12}$ fractions from petroleum distillation, such as xylene mixtures or substituted naphthalenes ; esters of phthalic acid, such as dibutyl or dioctyl phthalate ; aliphatic hydrocarbons, such as cyclohexane or the paraffins ; alcohols and glycols or esters thereof, such as ethanol, ethylene glycol, ethylene glycol monomethyl ether or ethylene glycol monoethyl ether ; ketones, such as cyclohexanone ; strongly polar solvents, such as N-methyl-2-pyrrolidone, dimethyl sulphoxide or N,N-dimethylformamide ; optionally epoxidized vegetable oils, such as epoxidized coconut oil or soybean oil ; and water.

Solid carriers, which may be used, for example, in dusts and dispersible powders, include natural mineral fillers, such as calcite, talc, kaolin, montmorillonite or attapulgite. In order to improve the physical properties of the composition, it is also possible to add highly dispersed silicic acid or highly dispersed absorbent polymers. Suitable granulated adsorptive carriers may be porous (such as pumice, ground brick, sepiolite or bentonite) or non-porous (such as calcite or sand). A wide variety of pregranulated materials, organic or inorganic, may also be used ; examples include dolomite and ground plant residues.

Surface-active agents which may be used are well known in the art and may be non-ionic, cationic or anionic agents having good emulsifying, dispersing and wetting properties. Mixtures of such agents may also be used.

Compositions may also contain stabilizers, anti-foaming agents, viscosity regulators, binders or adhesives or any combination thereof, as well as fertilizers or other active substances to achieve special effects.

Pesticidal compositions will generally contain : from 0.01 to 99 %, more preferably from 0.1 to 95 %, by weight of the active compound ; from 1 to 99.99 % of a solid or liquid additive ; and from 0 to 25 %, more preferably from 0.1 to 25 %, of a surface-active agent. Whereas commercial products are generally sold as concentrated compositions, they are generally diluted by the end-user to a concentration of from 0.001 to 0.000 1 % by weight (from 10 to 1 ppm).

The invention is further illustrated by the following Examples, of which Examples 1 to 6 illustrate the preparation of various compounds of the invention, Examples 7 to 12 illustrate the formulation of compositions of the invention and Examples 13 to 18 demonstrate the activity of a compound of the invention. In the Examples, all percentages are by weight.

## Example 1

5β-(3-carboxypropionyloxy) derivative of milbemycin D

111 mg of milbemycin D and 25 mg of 4-dimethylaminopyridine were dissolved in 2 ml of pyridine. 120 mg of succinic anhydride were added to the resulting solution, with ice-cooling (0-5 °C), and the mixture was allowed to react at room temperature for 24 hours. At the end of this time, the mixture was poured, with ice-cooling, into 2N hydrochloric acid and then extracted with diethyl ether. The organic extract was washed with water and dried over anhydrous magnesium sulphate. The solvent was then distilled off under reduced pressure. The resulting residue was purified by column chromatography through silica gel eluted with mixtures of benzene and ethyl acetate in volume ratios ranging from 10 : 5 to 4 : 6, to give 75 mg of the title compound, melting at 129-134 °C.

Infrared absorption spectrum
(Nujol-Trademark) $\upsilon_{max}$ $cm^1$ :
3 470, 3 200, 1 740, 1 720, 1 165.
Mass spectrum (m/e) :
656($M^+$), 428, 209.

## Example 2

5β-(3-carboxypropionyloxy) derivative of milbemycin D

By repeating the procedure described in Example 1.4 g of the title compound were prepared from 4.33 g of milbemycin D, 50 ml of pyridine, 4.68 g of succinic anhydride and 0.1 g of 4-dimethylaminopyridine.

## Example 3

Sodium salt of 5β-(3-carboxypropionyloxy) derivative of milbemycin D

The 5β-(3-carboxypropionyloxy) derivative of milbemycin D was reacted in aqueous solution with an equimolar amount of sodium bicarbonate, to give the desired salt melting at 185-190 °C (with decomposition).

## Example 4

5β-(4-carboxybutyryloxy) derivative of milbemycin D

The procedure described in Example 1 was repeated, except that glutaric anhydride was used in place of the succinic anhydride, to give the title compound, melting at 121-123 °C (with decomposition).

Example 5

5β-(3-carboxy-2-propenoyloxy) derivative of milbemycin D

The procedure described in Example 1 was repeated, except that the succinic anhydride was replaced by maleic anhydride, to give the title compound, melting at 132-135 °C.

Example 6

5β-(2-carboxybenzoyloxy) derivative of milbemycin D

The procedure described in Example 1 was repeated, except that the succinic anhydride was replaced by phthalic anhydride, to give the desired compound, melting at 152-155 °C.

Example 7

Wettable powders

The ingredients used were as follows :

| | (a) | (b) | (c) |
|---|---|---|---|
| active compound | 25 % | 50 % | 75 % |
| sodium lignin sulphonate | 5 % | 5 % | — |
| sodium lauryl sulphate | 3 % | — | 5 % |
| sodium diisobutyl-naphthalene sulphonate | — | 6 % | 10 % |
| octylphenol polyethylene glycol ether | — | 2 % | — |
| highly dispersed silicic acid | 5 % | 10 % | 10 % |
| kaolin | 62 % | 27 % | — |

In the polyethylene glycol ether used, the degree of polymerization of ethylene glycol was 7-8.

The active compound was well mixed with the other ingredients and then the mixture was ground thoroughly in a mill. Wettable powders, which can be diluted with water to give suspensions of the required concentration, were thereby produced.

Example 8

Emulsifiable concentrate

The ingredients used were as follows :

| | |
|---|---|
| active compound | 10 % |
| octylphenol polyethylene glycol ether (degree of polymerization 4-5) | 3 % |
| calcium dodecylbenzene sulphonate | 3 % |
| castor oil polyethylene glycol ether (degree of polymerization 36) | 4 % |
| cyclohexanone | 30 % |
| xylene mixture (commercial mixture of xylene isomers) | 50 % |

The ingredients were simply mixed, to give an emulsifiable concentrate, from which emulsions of the required concentration can be obtained by dilution with water.

Example 9

Dusts

The following ingredients were mixed and ground in a mill, to give dusts :

| | (a) | (b) |
|---|---|---|
| active compound | 5 % | 8 % |
| talc | 95 % | — |
| kaolin | — | 92 % |

Example 10

Extruded granulate

The ingredients used were as follows :

| | |
|---|---|
| active compound | 10 % |
| sodium lignin sulphonate | 2 % |
| carboxymethylcellulose | 1 % |
| kaolin | 87 % |

The active compound was mixed and ground with the other ingredients. The mixture was then moistened with water, after which it was extruded and dried in a stream of air.

Example 11

Coated granulate

The ingredients used were as follows :

| | |
|---|---|
| active compound | 3 % |
| polyethylene glycol (PEG 200) | 3 % |
| kaolin | 94 % |

The kaolin was moistened with the polyethylene glycol and then the finely ground active compound was evenly applied in a mixer to the moistened kaolin, to give dust-free coated granules.

Example 12

Suspension concentrate

The finely ground active compound was intimately mixed with the other ingredients in the following proportions :

| | |
|---|---|
| active compound | 40 % |
| ethylene glycol | 10 % |
| nonylphenol polyethylene glycol ether (degree of polymerization 15) | 6 % |
| sodium lignin sulphonate | 10 % |
| carboxymethylcellulose | 1 % |
| 37 % aqueous formaldehyde | 0.2 % |
| 75 % aqueous emulsion of silicone oil | 0.8 % |
| water | 32 % |

The resulting suspension concentrate can be diluted with water to give suspensions of the required concentration.

Example 13

Effect on migrating larve of Ascaris suum in mice

The animals employed in this test were white male mice, each weighing 25-30 g. The mice were divided into groups, each containing 5 animals, the groups being non-infected and untreated, infected and untreated, infected and treated with milbemycin D or infected and treated with the sodium salt of the 5β-(3)carboxypropionyloxy) derivative of milbemycin D (abbreviated as « Na succinate of milbemycin D »).

Each mouse in the infected groups was inoculated orally, by means of a stomach tube, with approximately 3 000 eggs of Ascaris suum (swine source).

24 hours after infection, each mouse in the treated groups was given orally a pharmaceutical composition containing milbemycin D or its sodium succinate derivative in an amount sufficient to provide 0.8 mg of the active compound per kg body weight. The composition contained 1.0 g of milbemycin D or its sodium succinate derivative, 0.1 g of butylated hydroxytoluene, 10 ml of dimethylacetamide and sufficient polyethylene glycol (PEG-400) to bring the total volume to 100 ml.

The same procedure was repeated exactly, except that the mice in the treated groups were given the same composition by subcutaneous injection.

7 days after infection, all the mice were killed and weighed and their lungs were examined for gross

lung lesions and for the number of larvae. The gross pathological pulminary lesions were graded according to the classification of Brown and Chan (1955, Am. J. Vet. Res., 16, 613-615). The larvae in the lungs were collected by means of a Baermann apparatus and counted. The results are reported in the following Tables as the percentage reduction in larvae in the lungs, calculated from the difference between the number of larvae in the lungs of infected untreated animals and infected treated animals, expressed as a percentage of the number in the lungs of infected untreated animals.

The weight gain over the test period of each animal was also calculated and is reported in the following Tables as the weight gain of each infected group, expressed as a percentage of the weight gain of the noninfected untreated group.

The results for oral administration are reported in Table 1 and the results for subcutaneous injection are reported in Table 2.

Table 1

Oral administration

| | Gross lung lesions | Weight gain (%) | % reduction in larvae |
|---|---|---|---|
| noninfected - untreated | 0 | 100 | — |
| infected - untreated | 4.2 | 76.5 | — |
| Milbemycin D | 3.4 | 78.8 | 50.9 |
| Na succinate of Milbemycin D | 2.8 | 86.2 | 78.4 |

Table 2

Subcutaneous injection

| | Gross lung lesions | Weight gain (%) | % reduction in larvae |
|---|---|---|---|
| noninfected - untreated | 0 | 100 | — |
| infected - untreated | 4.4 | 73.4 | — |
| Milbemycin D | 3.8 | 75.5 | 26.3 |
| Na succinate of Milbemycin D | 2.8 | 85.0 | 82.3 |

Example 14

Insecticidal activity against Spodoptera littoralis and Heliothis virescens

Cotton plants were sprayed with a solution containing 0.6, 0.8, 1.0, 1.2 or 1.4 ppm of the compound to be tested. After the coating had dried, the plants were populated with larvae of the species Spodoptera littoralis (L3-stage) or Heliothis virescens (L3-stage). Two plants were used for each test compound and each test species and a mortality count was made 2, 4, 24 and 48 hours after the larvae had been added. The test was carried out at 28 °C and 60 % relative humidity.

All of the compounds of Examples 1-6 effected a 100 % kill of larvae of both species within 24 hours at solution concentrations within the range of 0.8 to 1.2 ppm.

Example 15

Insecticidal contact activity against Myzus persicae

Before the start of the test, bean plants (Vicia faba) which had been reared in water were populated each with about 200 insects of the species Myzus persicae. 3 days later, the treated plants were sprayed from a distance of 30 cm, until they were dripping wet, with a solution containing 1.0, 1.5, 2.0, 2.5 or 3 ppm of the compound to be tested. Two plants were used for each test compound at each

concentration and a mortality count was made after a further 24 hours. At this time, all of the compounds of Examples 1-6 effected a 100 % kill of the insects at a concentration within the range from 1.5 to 2.5 ppm.

Example 16

Systemic insecticidal activity against Aphis craccivora

Bean plants which had grown roots were transplanted into pots each containing 600 $cm^3$ of soil, and then 50 ml of a solution containing 1.0, 1.5, 2.0, 2.5 or 3.0 ppm of the compound to be tested was poured directly onto the soil. After 24 hours, the parts of the plants above the soil were populated with lice of the species Aphis craccivora and a plastic cylinder was slipped over the plants, to protect the lice from any contact with the compound under test, either directly or through evaporation.

A mortality count was made 24 and 48 hours after the start of the test. Two plants, each in a separate pot, were used for each concentration of each test substance. The test was carried out at 25 °C and 70 % relative humidity.

Within the range of concentrations from 1.5 to 2.5 ppm, the compounds of Examples 1-6 had a 100 % systemic insecticidal activity against these insects.

Example 17

Activity against Aedes aegypti

The appropriate amount of a 0.01 % w/w solution in acetone of each compound to be tested was pipetted onto the surface of 150 ml of water in a beaker, to give a concentration of the active compound of 0.1, 0.2 or 0.3 ppm. After the acetone had evaporated, 2 day old larvae of the species Aedes aegypti were then put into each beaker (30 to 40 per beaker). A mortality count was made after 1, 2 and 5 days.

After 5 days, a 100 % kill of these larvae was achieved by all of the compounds of Examples 1-6 at concentrations within the range from 0.1 to 0.2 ppm.

Example 18

Acaricidal activity against Tetranychus urticae and Tetranychus cinnabarinus

The primary leaves of plants of the species Phaseolus vulgaris were infected by contact with an infested piece of leaf from a mass culture of acarids of the species Tetranychus urticae (organic phosphatesensitive) or Tetranychus cinnabarinus (organic phosphate-tolerant) ; tolerance is with respect to Diazinon. 16 hours after infection, the infested plants were sprayed, until dripping wet, with a test solution containing 0.2, 0.4, 0.6, 0.8 or 1.0 ppm of the compound to be tested. The plants were assessed after 24 hours and again after 7 days by examining imagos and larvae (all mobile stages) under a binocular microscope, to determine living and dead individuals. One plant was used for each concentration and each test compound. The plants were kept during the test in greenhouse compartments at 25 °C.

All of the compounds of Examples 1-6 achieved 100 % activity within the test period against acarids of the species Tetranychus urticae and Tetranychus cinnabarinus at concentrations within the range from 0.4 to 0.6 ppm.

Example 19

Acaricidal effects against Boophilus microplus

Rows, each containing 10 engorged female ticks of the species Boophilus microplus (strain Biarra), were stuck dorsally onto double-sided sticky tape spread out on polyvinyl chloride panels. The ticks were then injected with 1, 0.1 or 0.01 × $10^{-6}$ g of the active compound. They were then kept in an air-conditioned chamber at 28 °C and at 80 % relative humidity. The effectiveness of the compounds was judged by their $IR_{90}$, which is the dose rate preventing reproduction (i. e. larval hatching) in at least 9 out of 10 females within 30 days after treatment. The compounds of Examples 1-6 were all fully effective at a concentration of 0.1 × $10^{-6}$ g.

**Claims**

1. Compounds of formula (II) :

(See formula page 11)

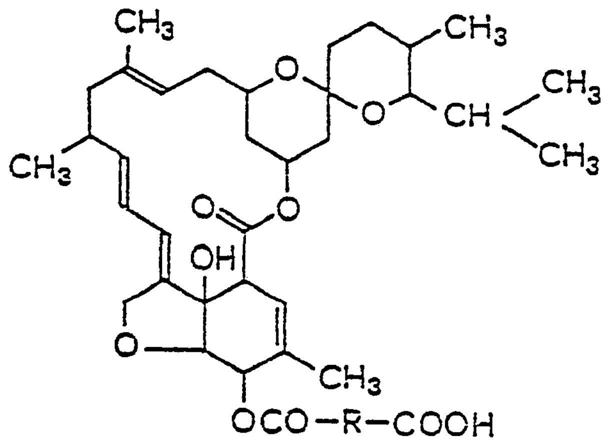

(II)

(wherein R represents an alkylene group having from 1 to 4 carbon atoms, a vinylene group or a group of formula :

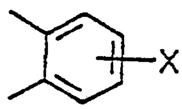

wherein X represents a hydrogen atom or an alkyl group having from 1 to 6 carbon atoms) and salts thereof.

2. Compounds as claimed in claim 1, wherein R represents an ethylene, trimethylene or vinylene group.

3. Compounds as claimed in claim 1, wherein R represents a group of formula :

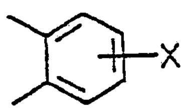

and X represents a hydrogen atom or a methyl, ethyl, propyl, butyl or isobutyl group.

4. Compounds as claimed in claim 3, wherein X represents a hydrogen atom or a methyl group.

5. Compounds as claimed in claim 4, wherein R represents an o-phenylene group.

6. Compounds as claimed in any one of claims 1 to 5, in the form of the sodium or potassium salt.

7. The 5β(3-carboxypropionyloxy) derivative of milbemycin D and its sodium salt.

8. An anthelmintic, acaricidal and insecticidal composition for pharmaceutical, veterinary, agricultural or horticultural use and comprising an anthelmintic, acaricidal and insecticidal compound in admixture with a carrier or diluent, wherein the compound is a compound as claimed in any one of claims 1 to 7.

9. A method of protecting non-human animals or plants from damage by acarids, helminths or insects, which comprises applying an active compound to said animals, seeds, said plants or to a locus including the same, wherein the compound is as claimed in any one of claims 1 to 7.

10. A process for producing a compound as claimed in any one of claims 1 to 7, by reacting milbemycin D with a dibasic acid of formula HOOC—R—COOH (in which R is as defined in any one of claims 1 to 5) or with a functional equivalent thereof, one carboxy group in said acid or equivalent being optionally protected, if necessary removing the protecting group and, if necessary, salifying the product.

11. A process as claimed in claim 10, in which said functional equivalent is a compound of formula (III) :

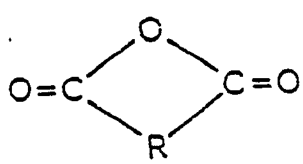

(III)

(in which R is as defined in claim 10).

12. A process as claimed in claim 10, in which said acid or said functional equivalent is a compound of formula (IV) :

Y—CO—R—COOH (IV)

(in which : R is as defined in any one of claims 1 to 5 ; Y represents a hydroxy group ; a halogen atom or a $C_2$-$C_5$ alkylcarboxy group ; and the group —COOH is optionally protected).

13. A process as claimed in claim 12, in which said compound of formula (IV) is a mono acid halide in which Y represents a halogen atom and in which the carboxy group is optionally protected.

**Patentansprüche**

1. Verbindungen der Formel (II)

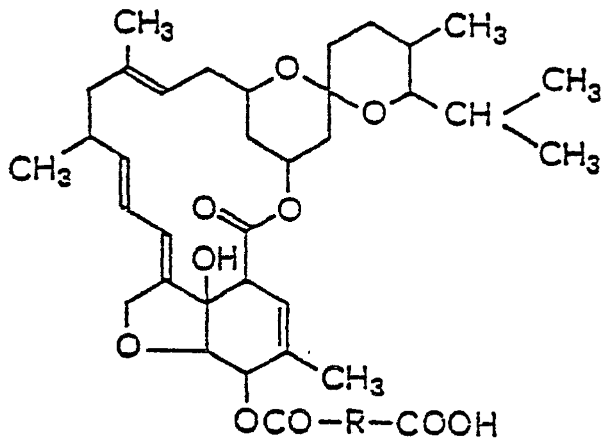

(II)

(worin R eine Alkylengruppe mit 1 bis 4 Kohlenstoffatomen, eine Vinylengruppe oder eine Gruppe der Formel

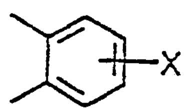

bedeutet, in der X ein Wasserstoffatom oder eine Alkylgruppe mit 1 bis 6 Kohlenstoffatomen darstellt) und deren Salze.

2. Verbindungen nach Anspruch 1, worin R eine Ethylen-, Trimethylen- oder Vinylengruppe bedeutet.

3. Verbindungen nach Anspruch 1, worin R eine Gruppe der Formel

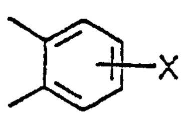

darstellt und X ein Wasserstoffatom oder eine Methyl-, Ethyl-, Propyl-, Butyl- oder Isobutylgruppe bedeutet.

4. Verbindungen nach Anspruch 3, worin X ein Wasserstoffatom oder eine Methylgruppe darstellt.

5. Verbindungen nach Anspruch 4, worin R eine o-Phenylengruppe darstellt.

6. Verbindungen nach einem der Ansprüche 1 bis 5 in Form des Natrium- oder Kaliumsalzes.

7. Das 5β-(3-Carboxypropionyloxy)-Derivat von Milbemycin D und dessen Natriumsalz.

8. Anthelmintische, acarizide und insektizide Zusammensetzung zur pharmazeutischen, veterinärmedizinischen, landwirtschaftlichen oder gartenwirtschaftlichen Anwendung, enthaltend eine anthelmintische, acarizide bzw. insektizide Verbindung im Gemisch mit einem Träger oder Verdünnungsmittel, wobei die Verbindung eine Verbindung gemäß einem der Ansprüche 1 bis 7 ist.

9. Verfahren zum Schützen von Tieren oder Pflanzen vor dem Befall durch Milben, Würmer oder Insekten, bei dem diese Tiere, Samen, diese Pflanzen oder ein Ort an dem diese sich befinden, mit einer aktiven Verbindung behandelt wird, wobei eine Verbindung nach einem der Ansprüche 1 bis 7 eingesetzt wird.

10. Verfahren zur Herstellung einer Verbindung gemäß einem der Ansprüche 1 bis 7 durch Umsetzen von Milbemycin D mit einer dibasischen Säure der Formel HOOC—R—COOH (in der R wie in einem der Ansprüche 1 bis 5 definiert ist) oder mit einem funktionellen Äquivalent davon, wobei eine Carboxygruppe in dieser Säure oder deren Äquivalent gegebenenfalls geschützt ist, erforderlichenfalls die Schutzgruppe entfernt und erforderlichenfalls das Produkt in das Salz übergeführt wird.

11. Verfahren nach Anspruch 10, bei dem das funktionelle Äquivalent eine Verbindung der Formel (III) ist :

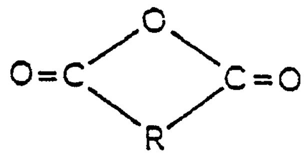

(III)

(worin R die Definition gemäß Anspruch 10 hat).

12. Verfahren nach Anspruch 10, bei dem die Säure oder deren funktionelles Äquivalent eine Verbindung der Formel (IV) ist :

Y—CO—R—COOH (IV)

(in der R wie in einem der Ansprüche 1 bis 5 definiert ist, Y eine Hydroxygruppe, eine Halogenatom oder eine $C_2$-$C_5$-Alkylcarboxygruppe darstellt und die Gruppe —COOH gegebenenfalls geschützt ist).

13. Verfahren nach Anspruch 12, bei dem die Verbindung der Formel (IV) ein Monocarbonsäurehalogenid ist, in dem Y ein Halogenatom bedeutet und in dem die Carboxygruppe gegebenenfalls geschützt ist.

**Revendications**

1. Composés de formule (II) :

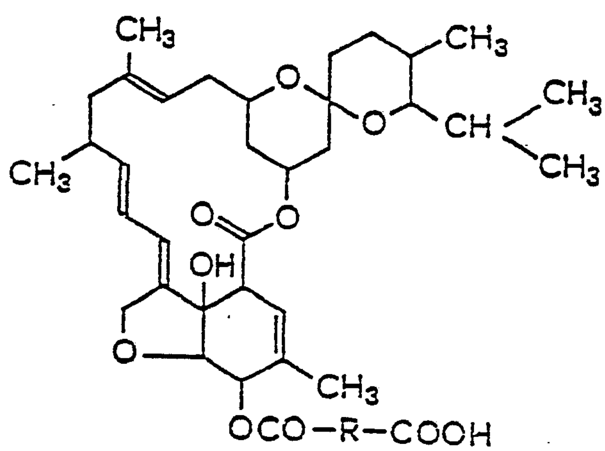

(II)

(dans laquelle R représente un groupe alkylène ayant de 1 à 4 atomes de carbone, un groupe vinylène ou un groupe de formule :

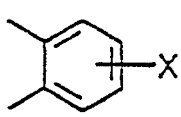

dans laquelle X représente un atome d'hydrogène ou un groupe alkyle ayant de 1 à 6 atomes de carbone) et leurs sels.

2. Composés selon la revendication 1, dans lesquels R représente un groupe éthylène, triméthylène ou vinylène.

3. Composés selon la revendication 1, dans lesquels R représente un groupe de formule :

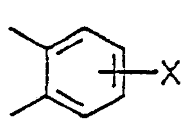

et X représente un atome d'hydrogène ou un groupe méthyle, éthyle, propyle, butyle ou isobutyle.

4. Composés selon la revendication 3, dans lesquels X représente un atome d'hydrogène ou un groupe méthyle.

5. Composés selon la revendication 4, dans lesquels R représente un groupe o-phénylène.

6. Composés selon l'une quelconque des revendications 1 à 5, sous forme du sel de sodium ou de potassium.

7. Dérivé 5β-(3-carboxypropionyloxy) de la milbémycine D et son sel de sodium.

8. Composition anthelmintique, acaricide et insecticide à usage pharmaceutique, vétérinaire, agricole ou horticole et comprenant un composé anthelmintique, acaricide et insecticide en mélange avec un véhicule ou diluant, dans laquelle le composé est un composé tel qu'il est défini dans l'une quelconque des revendications 1 à 7.

9. Procédé pour la protection d'organismes animaux non humains ou de plantes contre le dommage causé par des acariens, des helminthes ou des insectes, qui comprend l'application d'un composé actif sur lesdits animaux, sur des graines, sur lesdites plantes ou sur un lieu les renfermant, dans lequel le composé est tel qu'il est défini dans l'une quelconque des revendications 1 à 7.

10. Procédé pour la préparation d'un composé tel qu'il est défini dans l'une quelconque des revendications 1 à 7, par réaction de la milbémycine D avec un acide dibasique de formule HOOC—R—COOH (dans laquelle R est tel qu'il est défini dans l'une quelconque des revendications 1 à 5) ou avec un équivalent fonctionnel de celui-ci, un groupe carboxy dans ledit acide ou équivalent étant facultativement protégé, enlèvement si nécessaire du groupe protecteur et, si nécessaire, salification du produit.

11. Procédé selon la revendication 10, dans lequel ledit équivalent fonctionnel est un composé de formule (III) :

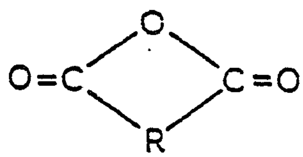

(III)

(dans laquelle R est tel qu'il est défini dans la revendication 10).

12. Procédé selon la revendication 10, dans lequel ledit acide ou ledit équivalent fonctionnel est un composé de formule (IV) :

$$Y—CO—R—COOH \quad (IV)$$

(dans laquelle R est tel qu'il est défini dans l'une quelconque des revendications 1 à 5 ; Y représente un groupe hydroxy, un atome d'halogène ou un groupe alkyl $(C_2-C_5)$-carboxy ; et le groupe —COOH est facultativement protégé).

13. Procédé selon la revendication 12, dans lequel ledit composé de formule (IV) est un halogénure de mono-acide dans lequel Y représente un atome d'halogène et dans lequel le groupe carboxy est facultativement protégé.